# EUROPEAN PATENT APPLICATION

(11) **EP 2 653 107 A1**
(43) Date of publication of application: **23.10.2013**
(21) Application number: 13155166.5
(22) Date of filing: 14.02.2013
(51) Int. Cl.: A61B 7/00, A61B 5/113, A61B 5/08

(54) **Apnea determining program, apnea determining device, and apnea determining method**

(30) Priority: 19.04.2012 JP 2012096052
(71) Applicant: FUJITSU LIMITED, Kawasaki-shi, Kanagawa 211-8588 (JP)
(72) Inventor: Nishida, Asako, Kawasaki-shi, Kanagawa 211-8588 (JP)
(74) Representative: Ward, James Norman

(57) **Abstract**

A portable terminal(1) includes a breath determining unit(31) that, when having detected sounds having similar frequency components periodically in picked-up sounds by a microphone while an object person is being asleep, determines the sounds to be breath sounds. The terminal includes a snore determining unit(32) that, when the sound pressure of the breath sounds is greater than a first level threshold value, determines the sounds to be a snoring sound. The terminal includes a body motion determining unit(34) that determines whether a body motion is detected after the breath sounds are determined. The terminal further includes an apnea determining unit(33) that determines whether a silent interval is detected from a period of time between timings of the breath sound and the body motion when the body motion is detected and that determines the silent interval to be an apnea state when the silent interval is detected.

## Description

### FIELD

The embodiment discussed herein is related to an apnea determining program, an apnea determining device, and an apnea determining method.

### BACKGROUND

In recent years, the number of patients with sleep apnea syndrome and the number of persons who will be potential sleep apnea syndrome patients have tended to increase every year. The sleep apnea syndrome is a disease that patient's symptom can be alleviated by early treatment. Therefore, it is important that the patients with sleep apnea syndrome and persons who will be potential sleep apnea syndrome patients are aware of sleep apnea.

In recent years, for example, a portable terminal has been developed which has a function of detecting sleep apnea. The portable terminal records the sound of an object person during sleep and detects a snoring sound from the recorded data. Then, when a silent interval is continuously detected between successive snoring sounds for a predetermined period of time, the portable terminal determines the silent interval to be an apnea state. The predetermined period of time is, for example, 10 seconds to 120 seconds. Therefore, the user of the portable terminal can recognize whether an object person is in an apnea state during sleep using the portable terminal.
Patent Document 1: Japanese Laid-open Patent Publication No. 2009-219713
Patent Document 2: Japanese National Publication of International Patent Application No. 11-505146
Patent Document 3: Japanese Laid-open Patent Publication No. 2006-167427

Examples of an apnea state generation pattern include a generation pattern "a snoring sound → apnea → a snoring sound" in which apnea occurs between successive snoring sounds and a generation pattern "a snoring sound → apnea → a body motion" in which, after a snoring sound is generated, apnea occurs and then a body motion is made due to apnea. In addition, as another generation pattern, there is the following generation pattern "a breath sound → apnea → a body motion" in which, after a breath sound, apnea occurs and a body motion is made due to apnea.

However, the above-mentioned portable terminal can respond to the generation pattern "a snoring sound → apnea → a snoring sound", but it is difficult for the portable terminal to respond to the generation pattern "a snoring sound → apnea → a body motion" or the generation pattern "a breath sound → apnea → a body motion". Therefore, it is difficult for the portable terminal to determine the apnea state from the generation pattern to which the portable terminal is not capable of responding.

Accordingly, it is an object in one aspect of an embodiment of the invention to provide an apnea determining program, an apnea determining device, and an apnea determining method capable of determining a state which can be considered as an apnea state with high accuracy.

### SUMMARY

According to an aspect of the embodiments, an apnea determining program causes a processor of an electronic apparatus to perform: when having detected sounds having similar frequency components periodically in picked-up sounds, first determining the detected sounds to be breath sounds of an object person; second determining whether a body motion of the object person is detected after the breath sounds are determined at the first determining; third determining whether a silent interval is detected from a period of time between timings of the breath sound determined at the first determining and the body motion detected by the second determining when the body motion is detected; and fourth determining the silent interval to be an apnea state when the silent interval is detected.

### BRIEF DESCRIPTION OF DRAWINGS

FIG. 1 is a diagram illustrating an example of a portable terminal according to this embodiment;
FIG. 2 is a diagram illustrating an example of the functional structure of a processor;
FIG. 3 is a diagram illustrating an example of the operation of a breath determining unit;
FIG. 4 is a diagram illustrating an example of the operation of a snore determining unit;
FIG. 5A is a diagram illustrating an example (a breath sound → apnea → a body motion) of the operation of an apnea determining unit;
FIG. 5B is a diagram illustrating an example (a snoring sound → apnea → a body motion) of the operation of the apnea determining unit;
FIG. 6 is a diagram illustrating an example (acoustic component) of the operation of a body motion determining unit;
FIG. 7 is a diagram illustrating an example (acceleration component) of the operation of the body motion determining unit;
FIG. 8 is a diagram illustrating an example of a sleep log screen of the portable terminal;
FIG. 9 is a flowchart illustrating an example of the processing operation of a processor in the portable terminal for a breathing determination process;
FIG. 10 is a flowchart illustrating an example of the processing operation of the processor in the portable terminal for a snoring determination process;
FIG. 11 is a flowchart illustrating an example of the processing operation of the processor in the portable terminal for a body motion determination process;
FIG. 12 is a flowchart illustrating an example of the processing operation of the processor in the portable terminal for a first apnea determination process;
FIG. 13 is a flowchart illustrating an example of the processing operation of the processor in the portable terminal for a second apnea determination process;
FIG. 14 is a flowchart illustrating an example of the processing operation of the processor in the portable terminal for a sleep advice providing process;
FIG. 15 is a flowchart illustrating an example of the processing operation of the processor in the portable terminal for a sleep environment determination process;
FIG. 16 is a flowchart illustrating an example of the processing operation of the processor in the portable terminal for a hospital site link process; and
FIG. 17 is a diagram illustrating an example of an electronic apparatus which executes an apnea determining program.

### DESCRIPTION OF EMBODIMENTS

Preferred embodiments of the present invention will be explained with reference to accompanying drawings. However, the disclosed technique is not limited by these embodiments.

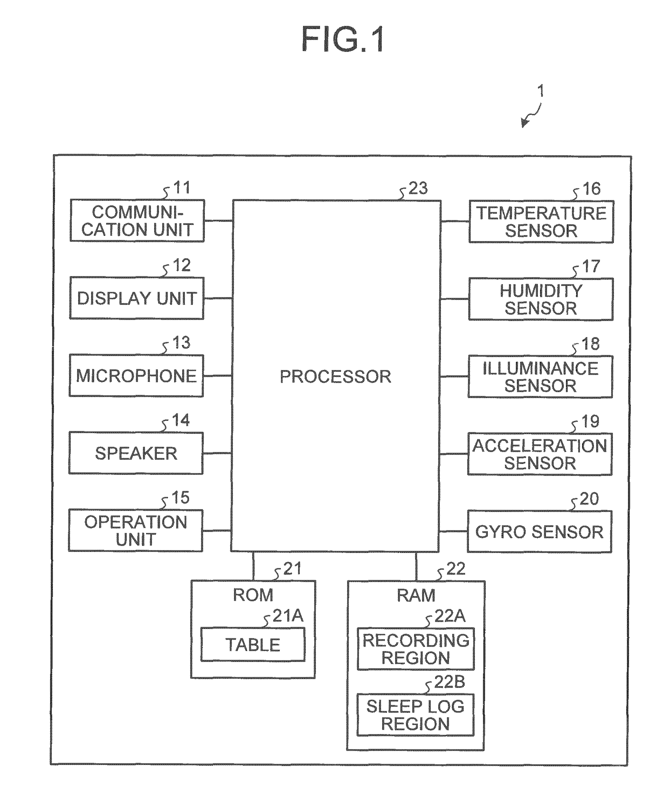

FIG. 1 is a diagram illustrating an example of a portable terminal according to an embodiment. A portable terminal 1 illustrated in FIG. 1 includes a communication unit 11, a display unit 12, a microphone 13, a speaker 14, an operation unit 15, a temperature sensor 16, a humidity sensor 17, an illuminance sensor 18, an acceleration sensor 19, and a gyro sensor 20. In addition, the portable terminal 1 includes a read only memory (ROM) 21, a random access memory (RAM) 22, and a processor 23.

The portable terminal 1 is, for example, a mobile phone such as a smart phone. The communication unit 11 is an interface with a wireless function. The display unit 12 is a liquid crystal display (LCD) which displays various kinds of information on a screen. The microphone 13 collects a sound such as a voice. The speaker 14 outputs a sound to the outside. The operation unit 15 is, for example, a touch panel device that detects an operation for an operation screen displayed on the display unit 12. The touch panel device is, for example, a resistance film type, but may be any type, such as a surface acoustic wave type, an infrared type, an electromagnetic induction type, or a capacitance type. The temperature sensor 16 measures, for example, the ambient temperature of the portable terminal 1. The humidity sensor 17 measures, for example, the ambient humidity of the portable terminal 1. The illuminance sensor 18 measures, for example, the ambient illuminance of the portable terminal 1.

The acceleration sensor 19 is a sensor that detects the acceleration of the portable terminal 1 along three axes, that is, the x-axis, the y-axis, and the z-axis. The gyro sensor 20 is a sensor which detects, for example, a three-axis angular speed. The ROM 21 is a storage unit that stores various kinds of programs including, for example, an apnea determining program. In addition, the ROM 21 stores a table 21A in which advices corresponding to various kinds of conditions, which will be described below, are stored. The RAM 22 stores various kinds of information. The RAM 22 includes a recording region 22A and a sleep log region 22B. The recording region 22A stores a sound during sleep which is collected by the microphone 13 as recorded data. The sleep log region 22B stores various kinds of sleep logs, which will be described below. The processor 23 controls the overall operation of the portable terminal 1.

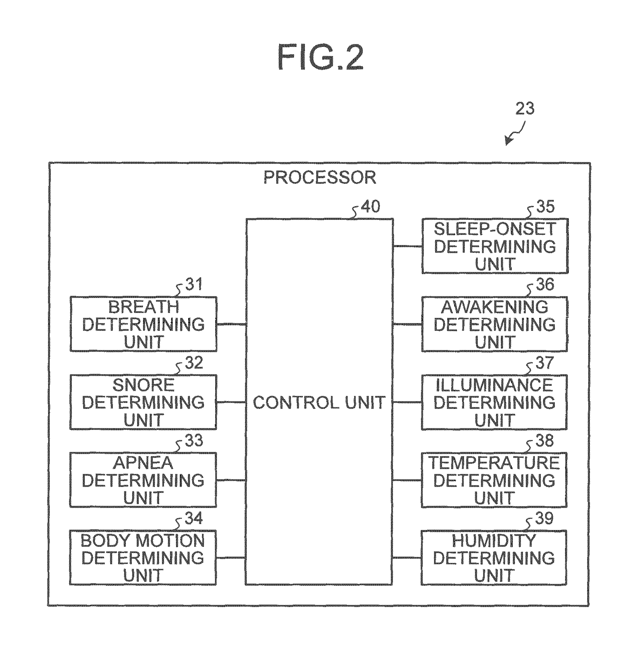

FIG. 2 is a diagram illustrating an example of the functional structure of the processor 23. The processor 23 reads the apnea determining program stored in the ROM 21 and forms various kinds of processes as functions on the basis of the read apnea determining program. The processor 23 illustrated in FIG. 2 has, as functions, a breath determining unit 31, a snore determining unit 32, an apnea determining unit 33, a body motion determining unit 34, a sleep-onset determining unit 35, an awakening determining unit 36, an illuminance determining unit 37, a temperature determining unit 38, a humidity determining unit 39, and a control unit 40.

The control unit 40 controls the overall operation of the processor 23. In addition, when a sleep log function, which will be described below, starts, the control unit 40 collects a sound through the microphone 13 and stores the collected sound as recorded data in the recording region 22A of the RAM 22. The sleep log function acquires various kinds of logs related to a breath sound, snoring, apnea, or a body motion in each sleep cycle.

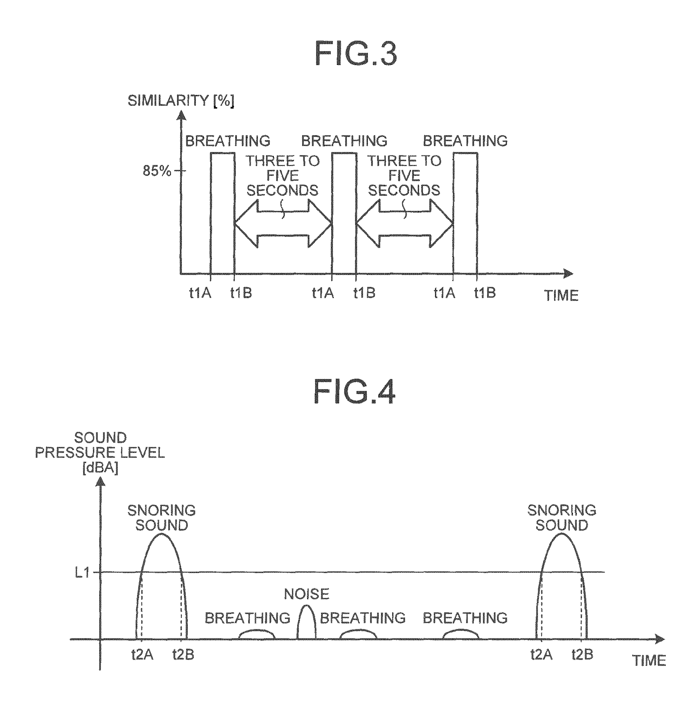

The breath determining unit 31 performs power spectrum analysis on the recorded data for one sleep cycle which is stored in the recording region 22A. Then, when a sound in which a component is similar at each frequency is periodically detected on the basis of the analysis result of the recorded data, the breath determining unit 31 determines that the sound is a breath sound of an object person, that is, the sound of breathing of the object person. The cycle varies depending on the object person. However, for convenience of explanation, it is assumed that the cycle is, for example, three seconds to five seconds. FIG. 3 is a diagram illustrating an example of the operation of the breath determining unit 31. The breath determining unit 31 sequentially determines the breath sounds from the recorded data on the basis of the similarity between the components of breath sound candidates at each frequency, considering that the component of the sound of breathing of the object person which is periodically generated during sleep at each frequency, that is, the component of the breath sound of the object person at each frequency is similar.

The breath determining unit 31 sequentially designates the component of a determination target at each frequency on the time axis of the recorded data. When the similarity between the component of the determination target at each frequency and the component of the breath sound candidate at each frequency is greater than 85%, the breath determining unit 31 determines that occurrence time of the sound of the component of the determination target at each frequency is the start timing t1A of the breath sound. When the similarity between the component of the breath sound at each frequency and the component of the breath sound candidate at each frequency is equal to or less than 85% after the start timing t1A of the breath sound is determined, the breath determining unit 31 determines that this is the end timing t1B of the breath sound. Then, the breath determining unit 31 stores the start time of the breath sound at the start timing t1A of the breath sound and the end time of the breath sound at the end timing t1B of the breath sound in the sleep log region 22B of the RAM 22.

The snore determining unit 32 determines whether the sound pressure level of the breath sound determined by the breath determining unit 31 is greater than a first level threshold value L1 in the recorded data for one sleep cycle. FIG. 4 is a diagram illustrating an example of the operation of the snore determining unit 32. When the sound pressure level of the breath sound is greater than the first level threshold value L1, the snore determining unit 32 determines that this is the start timing t2A of a snoring sound. When the breath sound is equal to or less than the first level threshold value L1 after the start timing t2A of the snoring sound is determined, the snore determining unit 32 determines that this is the end timing t2B of the snoring sound. It is assumed that the first level threshold value L1 is, for example, 40 dBA. Then, the snore determining unit 32 stores, in the sleep log region 22B, the start time of the snoring sound at the start timing t2A of the snoring sound, the end time of the snoring sound at the end timing t2B of the snoring sound, recorded data for the snoring sound, and the number of snores per hour in one sleep cycle.

Although a sound is detected at timing other than the timings of the breath sound determined by the breath determining unit 31 and the sound pressure level of the sound is greater than the first level threshold value L1, the snore determining unit 32 determines that the sound is noise. As a result, the snore determining unit 32 can prevent an error in the determination of the snoring sound due to noise which is generated between successive breath sounds.

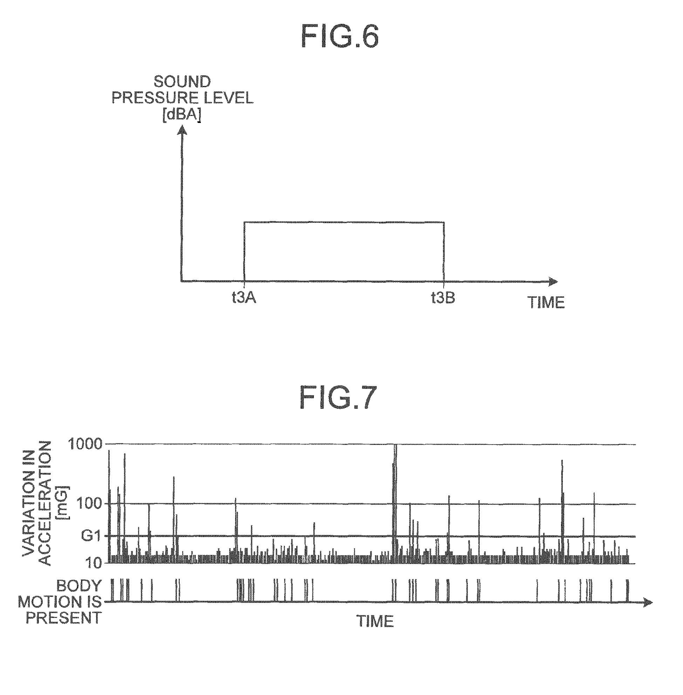

The body motion determining unit 34 determines a body motion, such as roll-over, on the basis of an acoustic component during sleep and an acceleration component during sleep. FIG. 6 is a diagram illustrating an example (acoustic component) of the operation of the body motion determining unit 34. The acoustic component in the body motion is a component of the sound at each frequency when a body motion, such as the rustling of bedclothes, is made during roll-over. The body motion determining unit 34 determines that there is a body motion when a sound component when the body motion is made is continuously detected from the recorded data for one sleep cycle for a predetermined period of time or more. Then, the body motion determining unit 34 stores the start time t3A and the end time t3B of the body motion and the number of body motions per hour in one sleep cycle in the sleep log region 22B.

FIG. 7 is a diagram illustrating an example (acceleration component) of the operation of the body motion determining unit 34. For the acceleration component during a body motion, when the object person rolls over, vibration is transmitted to the portable terminal 1 provided in the bed and is detected as a variation in acceleration. The unit of the variation in acceleration is mG. The body motion determining unit 34 determines that there is a body motion when the variation in acceleration during sleep which is obtained by the acceleration sensor 19 is greater than a body motion threshold value G1. Then, the body motion determining unit 34 stores the start time and end time of the body motion and the number of body motions per hour in one sleep cycle in the sleep log region 22B.

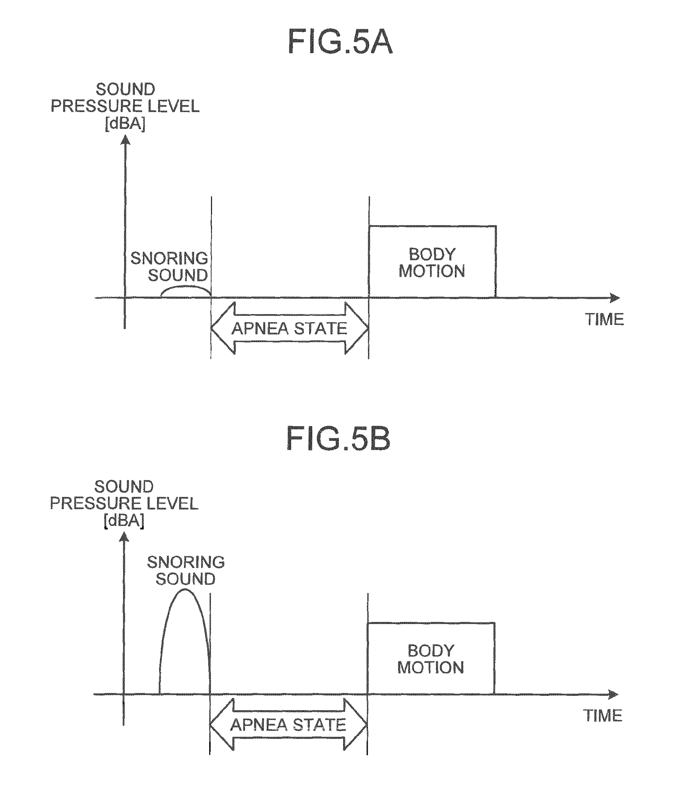

The apnea determining unit 33 designates the breath sound determined by the breath determining unit 31 and determines whether a body motion is detected after the designated breath sound. When the body motion is detected, the apnea determining unit 33 determines whether a silent interval is continuously detected for a predetermined period of time or more between the breath sound and the body motion. When the silent interval is detected between the breath sound and the body motion, the apnea determining unit 33 determines that the silent interval is an apnea state. In the silent interval, for example, a sound with a sound pressure level of less than 3 dB is maintained for ten seconds or more. FIG. 5A is a diagram illustrating an example (a breath sound → apnea → a body motion) of the operation of the apnea determining unit 33. When the silent interval is detected between the breath sound and the body motion, the apnea determining unit 33 determines that the silent interval is an apnea state. That is, as illustrated in FIG. 5A, the apnea determining unit 33 can determine central apnea with a pattern of a breath sound → apnea → a body motion. The apnea determining unit 33 stores the start time and end time of the apnea state and the number of apneas per hour in one sleep cycle in the sleep log region 22B.

The apnea determining unit 33 designates the snoring sound determined by the snore determining unit 32 and determines whether a body motion is detected after the designated snoring sound. When the body motion is detected, the apnea determining unit 33 determines whether the silent interval is continuously detected for a predetermined period of time or more between the snoring sound and the body motion. When the silent interval is detected between the snoring sound and the body motion, the apnea determining unit 33 determines that the silent interval is the apnea state. FIG. 5B is a diagram illustrating an example (a snoring sound → apnea → a body motion) of the operation of the apnea determining unit 33. When the silent interval is detected between the snoring sound and the body motion, the apnea determining unit 33 determines that the silent interval is the apnea state. That is, as illustrated in FIG. 5B, the apnea determining unit 33 can determine obstructive apnea with a pattern of a snoring sound → apnea → a body motion. The apnea determining unit 33 stores the start time and end time of the apnea state and the number of apneas per hour in one sleep cycle in the sleep log region 22B.

The sleep-onset determining unit 35 checks the continuity of the breath sound (the sound of breathing) determined by the breath determining unit 31 and determines whether the object person falls asleep using an AW2-type sleep determination algorithm. When the object person falls asleep, the sleep-onset determining unit 35 stores the sleep-onset time in the sleep log region 22B. The awakening determining unit 36 determines whether the object person is awake on the basis of the motion of the object person during sleep. Then, the awakening determining unit 36 stores the awakening time in the sleep log region 22B.

The illuminance determining unit 37 determines whether the current illuminance detected by the illuminance sensor 18 is within a comfortable illuminance threshold value. The comfortable illuminance threshold value is, for example, 200 lux. When the current illuminance is less than the comfortable illuminance threshold value, the control unit 40 reads an illuminance advice on comfortable sleep corresponding to the conditions from the table 21A and displays the read advice on the screen of the display unit 12. When the current illuminance is not less than the comfortable illuminance threshold value, the control unit 40 reads an illuminance advice on an illuminance change corresponding to the conditions from the table 21A and displays the read advice on the screen of the display unit 12.

The temperature determining unit 38 determines whether the current temperature detected by the temperature sensor 16 is within a comfortable temperature range. The comfortable temperature range is, for example, from 16°C to 20°C and may be changed depending on the season. When the current temperature is within the comfortable temperature range, the control unit 40 reads a temperature advice on comfortable sleep corresponding to the conditions from the table 21A and displays the read advice on the screen of the display unit 12. When the current temperature is beyond the comfortable temperature range, the control unit 40 reads a temperature advice on a temperature change corresponding to the conditions from the table 21A and displays the read advice on the screen of the display unit 12.

The humidity determining unit 39 determines whether the current humidity detected by the humidity sensor 17 is within a comfortable humidity range. The comfortable humidity range is, for example, from 45% to 55% and may be changed depending on the season. When the current humidity is within the comfortable humidity range, the control unit 40 reads a humidity advice on comfortable sleep corresponding to the conditions from the table 21A and displays the read advice on the screen of the display unit 12. When the current humidity is beyond the comfortable humidity range, the control unit 40 reads a humidity advice on a humidity change corresponding to the conditions from the table 21A and displays the read advice on the screen of the display unit 12.

The control unit 40 calculates the intensity of exercise on the basis of the acceleration data detected by the acceleration sensor 19 and calculates the amount of activity on the basis of the calculated intensity of exercise. Then, the control unit 40 stores the amounts of activity per hour, day, week, and month in the RAM 22.

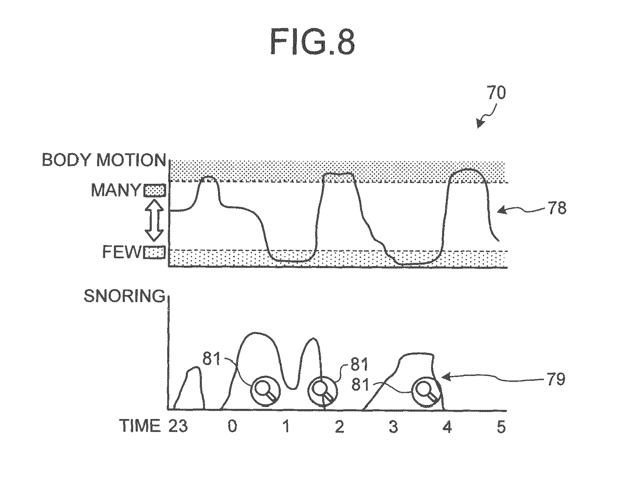

When an operation of tapping a graph button is detected, the control unit 40 displays a sleep log screen 70 illustrated in FIG. 8 having a graph indicating, for example, the number of body motions, the number of snores, or the number of apneas on the display unit 12.

When an operation of tapping a calendar button on a sleeping screen is detected, the control unit 40 displays the calendar screen on the display unit 12. When an operation of tapping an environment check button is detected, the control unit 40 displays a sleep environment screen indicating the current ambient illuminance, temperature, and humidity of the portable terminal 1 on the display unit 12. When an operation of tapping a sleep button on the sleeping screen is detected, the control unit 40 starts the sleep log function and displays a wake-up screen on the display unit 12. When an operation of tapping a wake-up button on the wake-up screen is detected, the control unit 40 recognizes wake-up.

The control unit 40 displays a sleep environment screen 60 on the display unit 12 on the basis of the determination results of the illuminance determining unit 37, the temperature determining unit 38, and the humidity determining unit 39. The sleep environment screen may include an illuminance advice, a temperature advice, a humidity advice, and a remeasurement button. The illuminance advice is a sleep advice based on the determination result of the current illuminance by the illuminance determining unit 37. The temperature advice is a sleep advice based on the determination result of the current temperature by the temperature determining unit 38. The humidity advice is a sleep advice based on the current humidity determined by the humidity determining unit 39. The remeasurement button is used to start the remeasurement of the current illuminance, humidity, and temperature.

The control unit 40 displays the sleep log screen 70 on the display unit 12 on the basis of the determination results of the breath determining unit 31, the snore determining unit 32, the apnea determining unit 33, and the body motion determining unit 34. The sleep log screen 70 illustrated in FIG. 8 may include the sleeping time at the designated date and time, sleep efficiency, a rank, bedtime, the wake-up time, the temperature and humidity with the passage of time, and sleep and awakening with the passage of time. The sleep log screen 70 includes a body motion number graph 78 with the passage of time, a snore number graph 79 with the passage of time, and an apnea mark 81.

The sleeping time is from the sleep-onset time to the wake-up time. The wake-up time is the time when the operation of tapping the wake-up button on the wake-up screen is detected or the time when the action of the object person is detected on the basis of the detection result of the acceleration sensor 19. The sleep efficiency is the ratio of the actual sleeping time obtained by subtracting the awakening time from the period from the sleeping time to the wake-up time to the period from the sleeping time to the wake-up time. The rank indicates the evaluation of the sleep efficiency. The sleeping time is, for example, the time when the operation of tapping the sleep button on the sleeping screen is detected. The wake-up time is, for example, the time when the operation of tapping the wake-up button on the wake-up screen is detected or the time when the action of the object person is detected on the basis of the detection result of the acceleration sensor 19. The temperature and humidity indicate the temperature and humidity per hour for the period from the sleeping time to the wake-up time. The sleep and awakening indicate a change in sleep and awakening from the sleeping time to the wake-up time. The sleep and awakening are distinguished by different colors.

The body motion number graph 78 indicates the number of body motions per hour for the period from the sleeping time to the wake-up time. The snore number graph 79 indicates the number of snores per hour for the period from the sleeping time to the wake-up time.

The apnea mark 81 indicates the time when the apnea state occurs on the snore number graph 79. When an operation of tapping the apnea mark 81 on the sleep log screen 70 is detected, the control unit 40 displays, on the screen of the display unit 12, an apnea detail screen on which the apnea state for the time indicated by the apnea mark is displayed. The sleep advice indicates a sleep advice suitable for conditions, which will be described below. A site button is used to access a designated site.

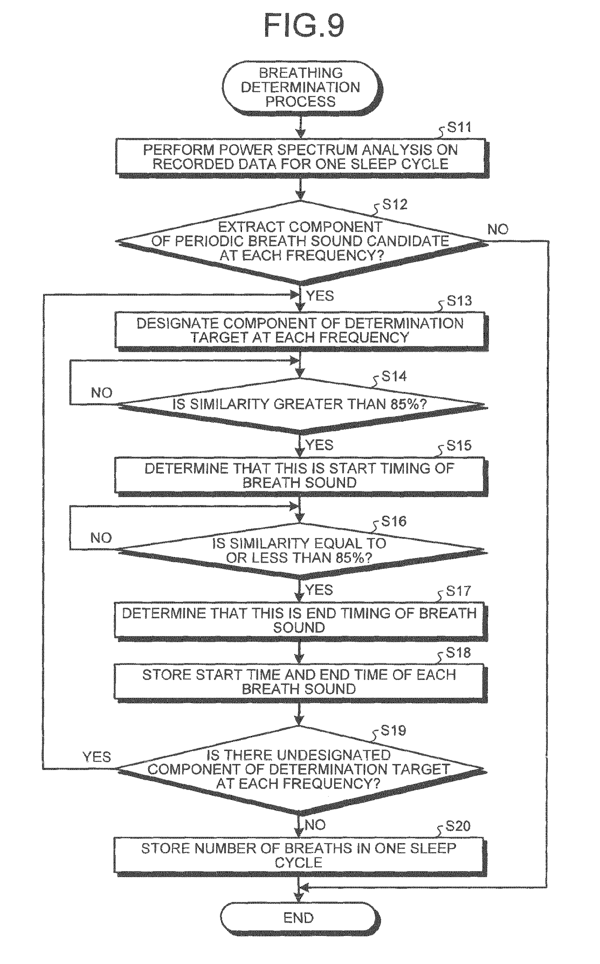

Next, the operation of the portable terminal 1 according to this embodiment will be described, FIG. 9 is a flowchart illustrating an example of the processing operation of the processor 23 in the portable terminal 1 for a breathing determination process. The breathing determination process determines a breath sound, which is the sound of breathing, from the recorded data for one sleep cycle. In FIG. 9, the breath determining unit 31 of the processor 23 reads the recorded data for one sleep cycle from the recording region 22A and performs power spectrum analysis on the read recorded data (Step S11). The breath determining unit 31 determines whether to extract the component of the breath sound candidate, which is the sound of breathing of the object person, at each frequency as a periodic sound having a similar component at each frequency from the recorded data on the basis of the result of the power spectrum analysis (Step S12). Then, when the component of the breath sound candidate at each frequency is extracted (Yes in Step S12), the breath determining unit 31 designates the component of the determination target at each frequency on the time axis in the recorded data (Step S13). The breath determining unit 31 sequentially designates the component of the determination target at each frequency on the time axis which is similar to the component of the breath sound candidate at each frequency. The breath determining unit 31 determines whether the similarity between the designated component of the determination target at each frequency and the component of the breath sound candidate at each frequency is greater than 85% (Step S14).

When the similarity between the designated component of the determination target at each frequency and the component of the breath sound candidate at each frequency is greater than 85% (Yes in Step S14), the breath determining unit 31 determines that this is the start timing of the breath sound (Step S15). In addition, after the start timing of the breath sound is determined, the breath determining unit 31 determines whether the similarity between the component of the determination target at each frequency and the component of the breath sound candidate at each frequency is equal to or less than 85% (Step S16). When the similarity between the component of the determination target at each frequency and the component of the breath sound candidate at each frequency is equal to or less than 85% (Yes in Step S16), the breath determining unit 31 determines that this is the end timing of the breath sound (Step S17). After the end timing of the breath sound is determined, the breath determining unit 31 stores the start time and end time of the breath sound in the sleep log region 22B (Step S18).

Then, the breath determining unit 31 determines whether there is an undesignated component of the determination target at each frequency (Step S19). When there is no undesignated component of the determination target at each frequency (No in Step S19), the breath determining unit 31 stores the number of breaths in one sleep cycle (Step S20) and ends the processing operation of the breathing determination process illustrated in FIG. 9.

In addition, when there is an undesignated component of the determination target at each frequency (Yes in Step S19), the breath determining unit 31 proceeds to Step S13, in order to designate the undesignated component of the determination target at each frequency. When it is difficult to extract the component of the breath sound candidate at each frequency (No in Step S12), the breath determining unit 31 ends the processing operation illustrated in FIG. 9. When the similarity between the component of the determination target at each frequency and the component of the breath sound candidate at each frequency is not greater than 85% (No in Step S14), the breath determining unit 31 proceeds to Step S14 in order to determine whether the similarity is greater than 85%. When the similarity between the component of the determination target at each frequency and the component of the breath sound candidate at each frequency is not equal to or less than 85% (No in Step S16), the breath determining unit 31 proceeds to Step S16 in order to determine whether the similarity is equal to or less than 85%.

In the breathing determination process illustrated in FIG. 9, the breath determining unit 31 extracts the component of a periodic breath sound candidate at each frequency from the recorded data for sleep and determines the timing when the similarity between the component of the determination target at each frequency and the component of the breath sound candidate at each frequency is greater than 85% to be the start timing of the breath sound. In addition, after the start timing of the breath sound is determined, the breath determining unit 31 determines the timing when the similarity between the component of the determination target at each frequency and the component of the breath sound candidate at each frequency is equal to or less than 85% to be the end timing of the breath sound. The breath determining unit 31 stores the start time and end time of the breath sound during sleep in the sleep log region 22B. As a result, the control unit 40 can recognize the start time and end time of each breath sound during sleep and the number of breaths per hour during sleep.

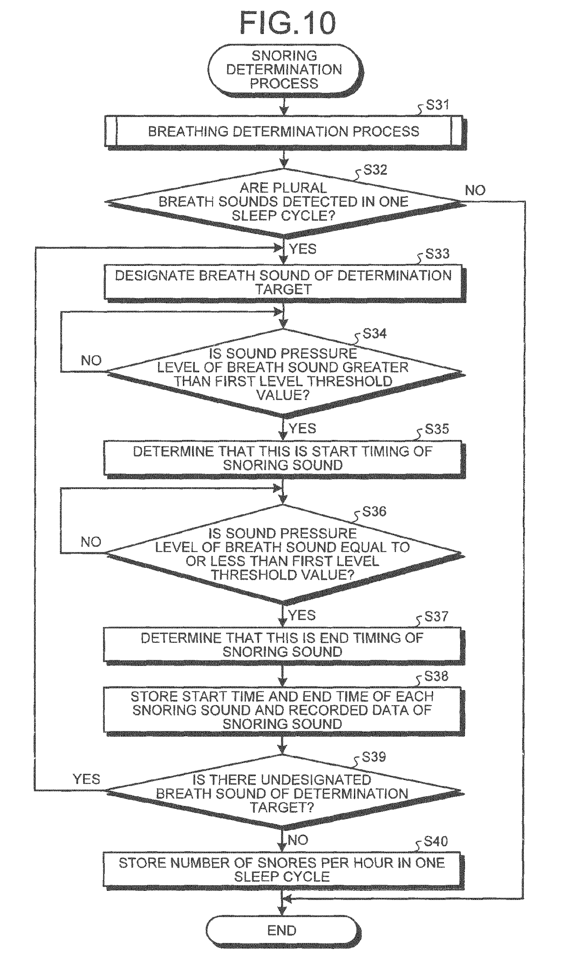

FIG. 10 is a flowchart illustrating an example of a processing operation of the processor 23 in the portable terminal 1 for a snoring determination process. The snoring determination process illustrated in FIG. 10 determines a snoring sound from the recorded data during one sleep. In FIG. 10, the snore determining unit 32 performs the breathing determination process illustrated in FIG. 9 (Step S31) and determines whether a plurality of breath sounds are detected in one sleep cycle (Step S32).

When the plurality of breath sounds are detected (Yes in Step S32), the snore determining unit 32 designates the breath sound of the determination target from the plurality of breath sounds (Step S33) and determines whether the sound pressure level of the breath sound of the determination target is greater than the first level threshold value (Step S34).

When the sound pressure level of the breath sound of the determination target is greater than the first level threshold value (Yes in Step S34), the snore determining unit 32 determines that this is the start timing of the snoring sound (Step S35). The snore determining unit 32 determines whether the sound pressure level of the breath sound of the determination target is equal to or less than the first level threshold value (Step S36). When the sound pressure level of the breath sound of the determination target is equal to or less than the first level threshold value (Yes in Step S36), the snore determining unit 32 determines that this is the end timing of the snoring sound (Step S37).

When the end timing of the snoring sound is determined, the snore determining unit 32 stores the start time and end time of the snoring sound and the recorded data for the snoring sound in the sleep log region 22B (Step S38). The snore determining unit 32 determines whether there is an undesignated breath sound of the determination target (Step S39). When there is no undesignated breath sound of the determination target (No in Step S39), the snore determining unit 32 stores the number of snores per hour in one sleep cycle in the sleep log region 22B (Step S40) and ends the processing operation illustrated in FIG. 10.

When there is an undesignated breath sound of the determination target (Yes in Step S39), the snore determining unit 32 proceeds to Step S33 in order to designate the breath sound of the determination target. When a plurality of breath sounds are not detected (No in Step S32), the snore determining unit 32 ends the processing operation illustrated in FIG. 10.

When the sound pressure level of the breath sound of the determination target is not greater than the first level threshold value (No in Step S34), the snore determining unit 32 proceeds to Step S34 in order to determine whether the sound pressure level is greater than the first level threshold value. When the sound pressure level of the breath sound of the determination target is not equal to or less than the first level threshold value (No in Step S36), the snore determining unit 32 proceeds to Step S36 in order to determine whether the sound pressure level is equal to or less than the first level threshold value.

In the snoring determination process illustrated in FIG. 10, the snore determining unit 32 determines that this is the start timing of the snoring sound when the sound pressure level of each breath sound during sleep which is determined by the breath determining unit 31 is greater than the first level threshold value. In addition, after the start timing of the snoring sound is determined, the snore determining unit 32 determines that this is the end timing of the snoring sound when the sound pressure level of the breath sound is equal to or less than the first level threshold value. The control unit 40 stores the start time and end time of each snoring sound during sleep in the sleep log region 22B. As a result, since the snore determining unit 32 determines the snoring sound with the timing of the breath sound determined by the breath determining unit 31, the accuracy of determining the snoring sound is improved. The control unit 40 can recognize the start time and end time of each snoring sound during sleep and the number of snores per hour during sleep.

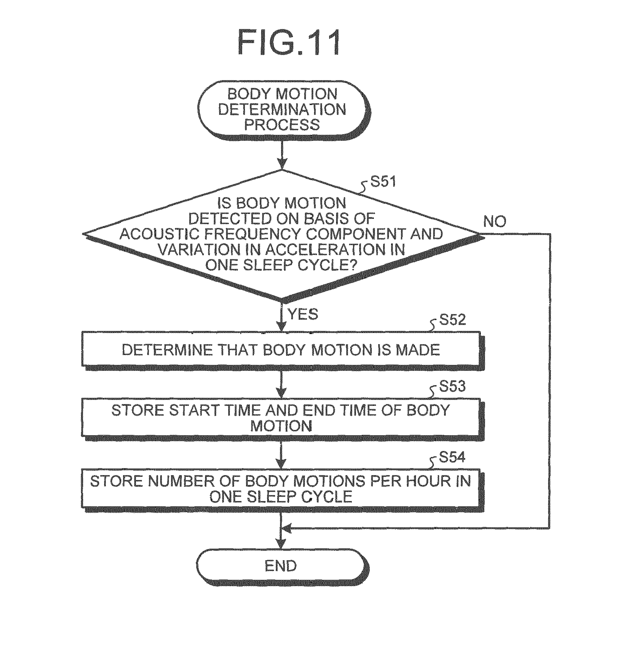

FIG. 11 is a flowchart illustrating an example of the processing operation of the processor 23 in the portable terminal 1 for a body motion determination process. The body motion determination process illustrated in FIG. 11 determines a body motion in one sleep cycle. In FIG. 11, the body motion determining unit 34 of the processor 23 determines whether a body motion is detected on the basis of the acoustic frequency component and the variation in acceleration in one sleep cycle (Step S51). The body motion determining unit 34 determines that a body motion is detected when a component of the sound which is generated by a body motion, such as the rustling of bedclothes, at each acoustic frequency for a predetermined period of time is detected from the recorded data for one sleep cycle. In addition, the body motion determining unit 34 determines that a body motion is detected when the variation in acceleration in one sleep cycle is greater than a body motion threshold value G1.

When a body motion is detected (Yes in Step S51), the body motion determining unit 34 determines that the body motion of the object person is made during sleep (Step S52). When it is determined that the body motion is made, the body motion determining unit 34 stores the start time and end time of the body motion in the sleep log region 22B (Step S53). The body motion determining unit 34 stores the number of body motions per hour in one sleep cycle in the sleep log region 22B (Step S54) and ends the processing operation illustrated in FIG. 11.

When no body motion is detected (No in Step S51), the body motion determining unit 34 ends the processing operation illustrated in FIG. 11.

In the body motion determination process illustrated in FIG. 11, the body motion determining unit 34 determines whether there is a body motion on the basis of the detection of the component of the sound which is generated by the body motion, such as the rustling of bedclothes, at each frequency from the recorded data during sleep. In addition, the body motion determining unit 34 determines that there is a body motion when the variation in the acceleration of vibration which is transmitted to the portable terminal 1 during sleep is greater than the body motion threshold value G1. Then, the body motion determining unit 34 stores the start time and end time of the body motion during sleep and the number of body motions per hour during sleep in the sleep log region 22B. As a result, the control unit 40 can recognize the start time and end time of the body motion during sleep and the number of body motions per hour during sleep.

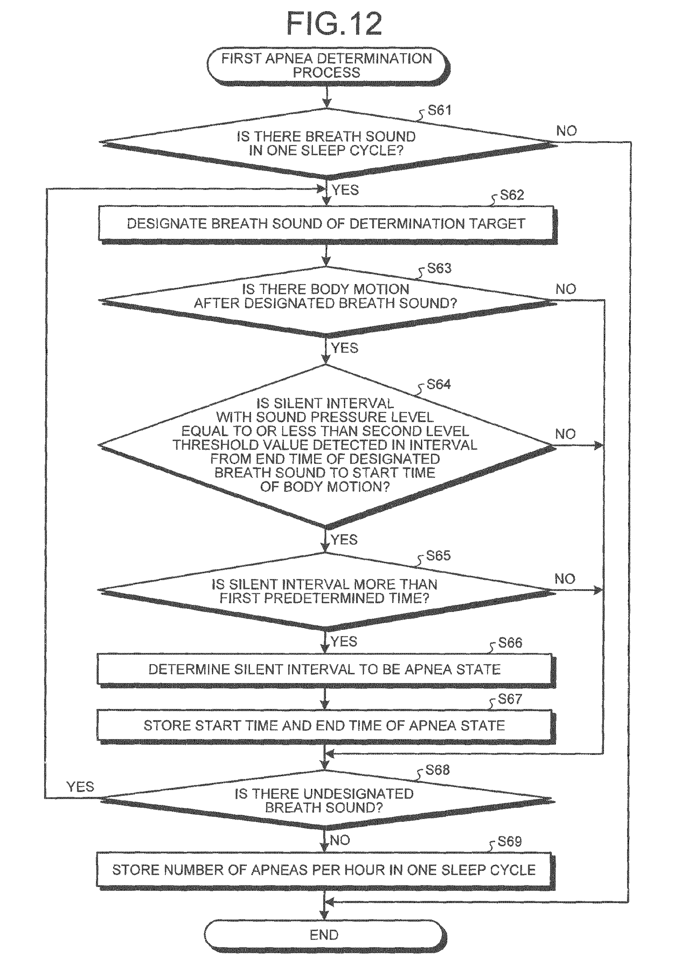

FIG. 12 is a flowchart illustrating an example of the processing operation of the processor 23 in the portable terminal 1 for a first apnea determination process. The first apnea determination process illustrated in FIG. 12 determines the apnea state between the snoring sound and the body motion when the body motion is detected after the snoring sound. In FIG. 12, the apnea determining unit 33 of the processor 23 determines whether there is a breath sound in one sleep cycle (Step S61). When there is a breath sound in one sleep cycle (Yes in Step S61), the apnea determining unit 33 designate the breath sound of the determination target (Step S62). When the breath sound of the determination target is designated, the apnea determining unit 33 determines whether a body motion is detected after the designated breath sound (Step S63). When a body motion is detected after the designated breath sound (Yes in Step S63), the apnea determining unit 33 determines whether a silent interval with a sound pressure level equal to or less than a second level threshold value is detected in a section from the end time of the designated breath sound to the start time of the body motion (Step S64). The second level threshold value is, for example, 3 dB.

When the silent interval with a sound pressure level equal to or less than the second level threshold value is detected in the section from the end time of the breath sound to the start time of the body motion (Yes in Step S64), the apnea determining unit 33 determines whether the duration of the silent interval is more than a first predetermined time (Step S65). The first predetermined time is, for example, 10 seconds, but may be appropriately changed. When the duration of the silent interval is more than the first predetermined time (Yes in Step S65), the apnea determining unit 33 determines the silent interval to be the apnea state (Step S66). The apnea determining unit 33 stores the start time and end time of the apnea state in the sleep log region 22B (Step S67).

The apnea determining unit 33 determines whether there is an undesignated breath sound of the determination target (Step S68). When there is no undesignated breath sound of the determination target (No in Step S68), the apnea determining unit 33 stores the number of apneas per hour in one sleep cycle in the sleep log region 22B (Step S69) and ends the processing operation illustrated in FIG. 12.

When there is an undesignated breath sound of the determination target (Yes in Step S68), the apnea determining unit 33 proceeds to Step S62 in order to designate the breath sound of the determination target. When there is no breath sound in one sleep cycle (No in Step S61), the apnea determining unit 33 ends the processing operation illustrated in FIG. 12. When no body motion is detected after the breath sound (No in Step S63), the apnea determining unit 33 proceeds to Step S68 in order to determine whether there is an undesignated breath sound of the determination target. When the silent interval with a sound pressure level equal to or less than the second level threshold value is not detected in the section from the end time of the breath sound to the start time of the body motion (No in Step S64), the apnea determining unit 33 proceeds to Step S68 in order to determine whether there is an undesignated breath sound of the determination target.

When the duration of the silent interval is not greater than the first predetermined time (No in Step S65), the apnea determining unit 33 proceeds to Step S68 in order to determine whether there is an undesignated breath sound of the determination target.

In the first apnea determination process illustrated in FIG. 12, when the silent interval is detected in the section from the breath sound to the body motion of the determination target, the apnea determining unit 33 determines the silent interval to be the apnea state. The apnea determining unit 33 stores the start time and end time of each apnea state with a pattern of a breath sound → apnea → a body motion and the number of apneas per hour during sleep in the sleep log region 22B. As a result, the control unit 40 can recognize the start time and end time of each apnea state with the pattern of a breath sound → apnea → a body motion and the number of apneas per hour during sleep. According to the first apnea determination process described above, the control unit 40 determines the apnea state on the basis of the breath sound and the body motion of the object person. Therefore, it is possible to detect central apnea.

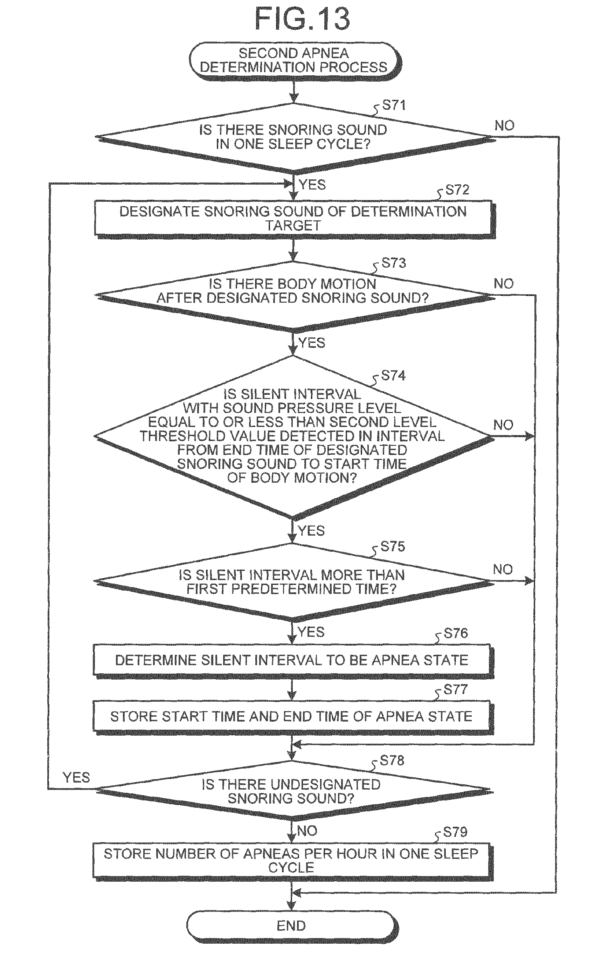

FIG. 13 is a flowchart illustrating an example of the processing operation of the processor 23 in the portable terminal 1 for a second apnea determination process. The second apnea determination process illustrated in FIG. 13 determines the apnea state in the section from the snoring sound to the body motion. In FIG. 13, the apnea determining unit 33 of the processor 23 determines whether there is a snoring sound in one sleep cycle (Step S71). When there is a snoring sound in one sleep cycle (Yes in Step S71), the apnea determining unit 33 designates the snoring sound of the determination target (Step S72). The apnea determining unit 33 determines whether a body motion is detected after the designated snoring sound (Step S73). When a body motion is detected after the designated snoring sound (Yes in Step S73), the apnea determining unit 33 determines whether a silent interval with a sound pressure level equal to or less than the second level threshold value is detected in the section from the end time of the designated snoring sound to the start time of the body motion (Step S74).

When the silent interval with a sound pressure level equal to or less than the second level threshold value is detected (Yes in Step S74), the apnea determining unit 33 determines whether the duration of the silent interval is more than the first predetermined time (Step S75). When the duration of the silent interval is more than the first predetermined time (Yes in Step S75), the apnea determining unit 33 determines the silent interval to be the apnea state (Step S76). The apnea determining unit 33 stores the start time and end time of the apnea state in the sleep log region 22B (Step S77).

The apnea determining unit 33 determines whether there is an undesignated snoring sound of the determination target (Step S78). When there is no undesignated snoring sound of the determination target (No in Step S78), the apnea determining unit 33 stores the number of apneas in one sleep cycle in the sleep log region 22B (Step S79) and ends the processing operation illustrated in FIG. 13.

When there is an undesignated snoring sound of the determination target (Yes in Step S78), the apnea determining unit 33 proceeds to Step S72 in order to designate the snoring sound of the determination target. When there is no snoring sound in one sleep cycle (No in Step S71), the apnea determining unit 33 ends the processing operation illustrated in FIG. 13. When a body motion is not detected after the snoring sound (No in Step S73), the apnea determining unit 33 proceeds to Step S78 in order to determine whether there is an undesignated snoring sound of the determination target. When the silent interval with a sound pressure level equal to or less than the second level threshold value is detected in the section from the snoring sound to the body motion (No in Step S74), the apnea determining unit 33 proceeds to Step S78 in order to determine whether there is an undesignated snoring sound of the determination target.

When the duration of the silent interval is not greater than the first predetermined time (No in Step S75), the apnea determining unit 33 proceeds to Step S78 in order to determine whether there is an undesignated snoring sound of the determination target.

In the second apnea determination process illustrated in FIG. 13, when a silent interval is detected in the section from the snoring sound to the body motion, the apnea determining unit 33 determines the silent interval to be the apnea state. The apnea determining unit 33 stores the start time and end time of each apnea state with a pattern of a snoring sound → apnea → a body motion and the number of apneas per hour during sleep in the sleep log region 22B. As a result, the control unit 40 can recognize the start time and end time of each apnea state with the pattern of a snoring sound → apnea → a body motion and the number of apneas per hour during sleep. According to the second apnea determination process described above, the control unit 40 determines the apnea state on the basis of the snoring sound and the body motion of the object person. Therefore, it is possible to detect obstructive apnea.

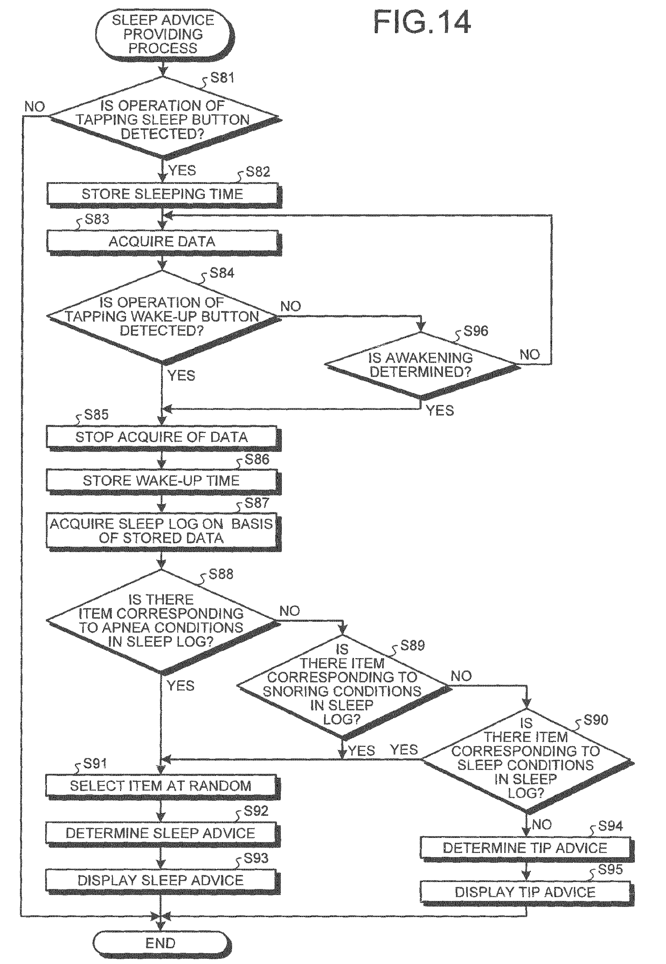

FIG. 14 is a flowchart illustrating an example of the processing operation of the processor 23 in the portable terminal 1 for a sleep advice providing process. The sleep advice providing process illustrated in FIG. 14 displays an advice corresponding to the content of the sleep log on the screen of the display unit 12. In FIG. 14, the control unit 40 of the processor 23 determines whether an operation of tapping the sleep button on the sleeping screen is detected (Step S81). When the operation of tapping the sleep button is detected (Yes in Step S81), the control unit 40 stores the sleeping time in the sleep log region 22B (Step S82). The control unit 40 acquires various kinds of data for the sleep log (Step S83). The various kinds of data for the sleep log include, for example, recorded data for the sound during sleep which is collected by the microphone 13 and data of a variation in acceleration which is collected by the acceleration sensor 19.

The control unit 40 determines whether an operation of tapping the wake-up button on the wake-up screen is detected (Step S84). When the operation of tapping the wake-up button is detected (Yes in Step S84), the control unit 40 stops the operation of acquiring data for the sleep log (Step S85) and stores the wake-up time in the sleep log region 22B (Step S86). The control unit 40 acquires the sleep log on the basis of the various kinds of data stored in the RAM 22 (Step S87). The sleep log is data obtained by, for example, the breathing determination process, the snoring determination process, the body motion determination process, the first apnea determination process, and the second apnea determination process. For example, the sleep log includes various kinds of data, such as the start time and end time of each breath sound, the start time and end time of each snoring sound, the number of snores, the start time and end time of each body motion, the number of body motions, the start time and end time of each apnea state, and the number of apneas.

The control unit 40 determines whether there is an item corresponding to apnea conditions in the sleep log (Step S88). The item corresponding to the apnea conditions is, for example, a condition item using the number of apneas. The control unit 40 selects a sleep advice corresponding to the apnea conditions from the table 21A. For example, when the apnea condition is that the number of apneas per hour in one sleep cycle is five or more, the control unit 40 selects a sleep advice "Do you often fall asleep? If so, please, click the following link button. <<Click here>>" from the table 21A. In addition, when the apnea condition is that a body motion is made within, for example, 30 seconds after apnea occurs and the total number of body motions during sleep is 10 or more, the control unit 40 selects a sleep advice "Since there are many body motions, you may not sleep deeply. If so, please, click the following link button. <<Click here>>" from the table 21A. When the apnea condition is that weight is 80 Kg or more, the number of snores is 20 or more, and the number of apneas is 20 or more, the control unit 40 selects a sleep advice "Do you know sleep apnea syndrome? A slightly obese person is likely to have sleep apnea syndrome" from the table 21A. For the conditions, such as weight, vital data, such as the weight, height, blood pressure, or pulse of the user, is registered in a designated site and the conditions, such as weight, are acquired from the designated sited.

When there is no item corresponding to the apnea conditions in the sleep log (No in Step S88), the control unit 40 determines whether there is an item corresponding to snoring conditions in the sleep log (Step S89). The item corresponding to the snoring conditions is, for example, a condition item using the number of snores. The control unit 40 selects a sleep advice corresponding to the snoring conditions from the table 21A. For example, when the snoring condition is that the number of snores is one or more in one sleep cycle, the control unit 40 selects a sleep advice "It seemed that a snoring sound was heard yesterday. Did you know it?" from the table 21A. For example, when the snoring condition is that the number of snores is 10 or more in one sleep cycle and the total duration of the snoring sound is 60 minutes or more in one sleep cycle, the control unit 40 selects, for example, a sleep advice "It seemed that a snoring sound was heard" from the table 21A.

When there is no item corresponding to the snoring conditions in the sleep log (No in Step S89), the control unit 40 determines whether there is an item corresponding to the sleep conditions (Step S90). The item corresponding to the sleep conditions is a condition item related to sleep, for example, a condition item using the number of awakenings. The control unit 40 selects a sleep advice corresponding to the sleep conditions from the table 21A. For example, when the sleep condition is that the sleeping time is less than five hours and the number of awakenings during sleep is five or more, the control unit 40 selects a sleep advice "Did you wake up several times in the night?. If so, please, click the following link button. <<Click here>>" from the table 21A. For example, when the sleep condition is that the period from the sleeping time to the sleep-onset time is 60 minutes or more and the total amount of activity per day is 1.0 EX or less, the control unit 40 selects a sleep advice "It takes a long time for you to fall asleep after you get into bed. You need to exercise to further increase the amount of activity per day." from the table 21A. For example, when the sleep condition is that the sleeping time is four hours or less for three successive days, the control unit 40 selects a sleep advice "Recently, the sleeping time has not been sufficient. Insufficient sleep is a great enemy to a diet!" from the table 21A.

When there is an item corresponding to the apnea conditions (Yes in Step S88), the control unit 40 selects the item corresponding to the conditions at random (Step S91). When there is a plurality of items corresponding to the conditions, the control unit 40 selects one item at random from the plurality of items. When there is an item corresponding to the snoring conditions (Yes in Step S89), or when there is an item corresponding to the sleep conditions (Yes in Step S90), the control unit 40 proceeds to Step S91 in order to select the item corresponding to the conditions at random. The control unit 40 determines the sleep advice corresponding to the randomly selected item from the table 21A (Step S92), displays the determined sleep advice on the screen of the display unit 12 (Step S93), and ends the processing operation illustrated in FIG. 14.

When there is no item corresponding to the sleep conditions in the sleep log (No in Step S90), the control unit 40 determines a tip advice from the table 21A (Step S94). The tip advice introduces knowledge for obtaining comfortable sleep and is, for example, an advice with content indicating "Sleep types include REM sleep and non-REM sleep. The REM sleep is deep sleep and the non-REM sleep is shallow sleep". Then, the control unit 40 displays the determined tip advice on the screen of the display unit 12 (Step S95) and ends the processing operation illustrated in FIG. 14.

When an operation of tapping a sleep button is not detected (No in Step S81), the control unit 40 ends the processing operation illustrated in FIG. 14. In addition, when an operation of tapping the wake-up button is not detected (No in Step S84), the awakening determining unit 36 determines whether awakening is detected by the wave-up behavior of the object person (Step S96).

When awakening is determined by the wave-up behavior of the object person (Yes in Step S96), the awakening determining unit 36 proceeds to Step S85 in order to stop the operation of acquiring data for the sleep log. When awakening is not determined by the wave-up behavior of the object person (No in Step S96), the awakening determining unit 36 proceeds to Step S83 in order to continuously perform the operation of acquiring data for the sleep log.

In the sleep advice providing process illustrated in FIG. 14, when the sleep log from the detection of the operation of tapping the sleep button to wake-up corresponds to the apnea conditions, the sleep advice corresponding to the apnea conditions is selected and the selected sleep advice is displayed on the screen of the display unit 12. As a result, the user can see the sleep advice and recognize the occurrence of apnea during sleep.

In the sleep advice providing process, when the sleep log from the detection of the operation of tapping the sleep button to wake-up corresponds to the snoring conditions, the sleep advice corresponding to the snoring conditions is selected and the selected sleep advice is displayed on the screen of the display unit 12. As a result, the user can see the sleep advice on snoring and recognize the occurrence of snoring during sleep.

In the sleep advice providing process, when the sleep log from the detection of the operation of tapping the sleep button to wake-up corresponds to the sleep conditions, the sleep advice corresponding to the sleep conditions is selected and the selected sleep advice is displayed on the screen of the display unit 12. As a result, the user can see the sleep advice and recognize disturbed sleep.

In the sleep advice providing process, when the sleep log from the detection of the operation of tapping the sleep button to wake-up does not correspond to any of apnea, the snoring conditions, and the sleep conditions, the tip advice on sleep is selected and the selected tip advice is displayed on the screen of the display unit 12. As a result, the user can see the tip advice and obtain knowledge of sleep.

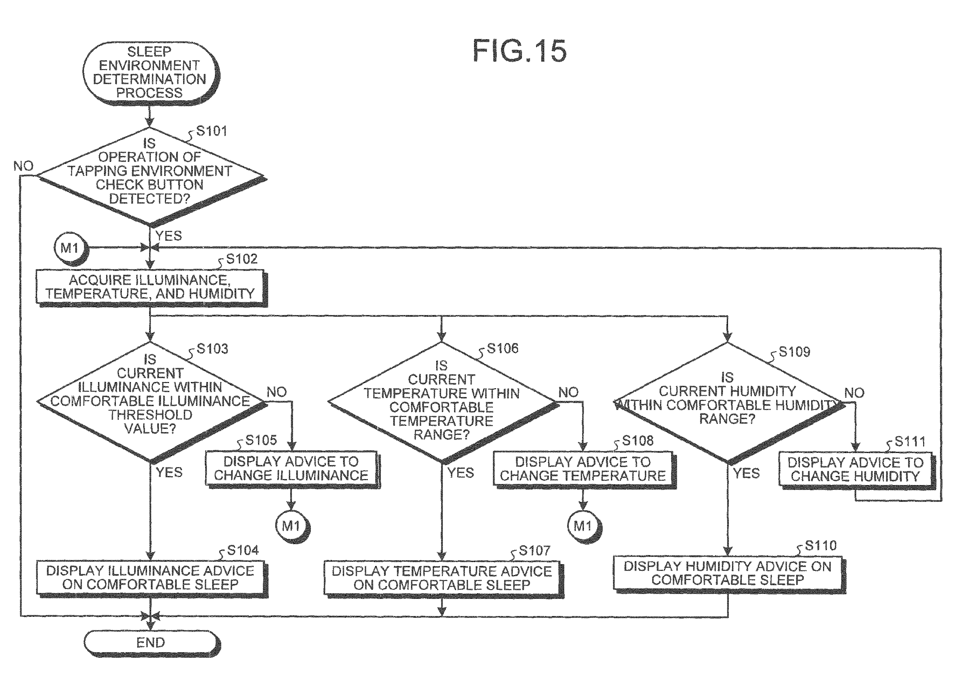

FIG. 15 is a flowchart illustrating an example of the processing operation of the processor 23 in the portable terminal 1 for a sleep environment determination process. The sleep environment determination process illustrated in FIG. 15 displays an environment advice corresponding to the current sleep environment on the screen of the display unit. In FIG. 15, the control unit 40 of the processor 23 determines whether an operation of tapping the environment check button is detected (Step S101). When the operation of tapping the environment check button is detected (Yes in Step S101), the control unit 40 acquires illuminance, temperature, and humidity through the illuminance sensor 18, the temperature sensor 16, and the humidity sensor 17, respectively (Step S102).

After the illuminance, temperature, and humidity are acquired in Step S102, the illuminance determining unit 37 determines whether the current illuminance is within a comfortable illuminance threshold value suitable for sleep (Step S103). When the current illuminance is within the comfortable illuminance threshold value (Yes in Step S103), the illuminance determining unit 37 displays an illuminance advice on comfortable sleep on the screen of the display unit 12 (Step S104) and ends the processing operation illustrated in FIG. 15. When the current illuminance is not within the comfortable illuminance threshold value (No in Step S103), the illuminance determining unit 37 displays an illuminance advice to change the illuminance on the screen of the display unit 12 (Step S105) and proceeds to Step S102 of M1 in FIG. 15 in order to acquire illuminance, temperature, and humidity.

After the illuminance, temperature, and humidity are acquired in Step S102, the temperature determining unit 38 determines whether the current temperature is within a comfortable temperature range suitable for sleep (Step S106). When the current temperature is within the comfortable temperature range (Yes in Step S106), the temperature determining unit 38 displays a temperature advice on comfortable sleep on the screen of the display unit 12 (Step S107) and ends the processing operation illustrated in FIG. 15. When the current temperature is not within the comfortable temperature range (No in Step S106), the temperature determining unit 38 displays a temperature advice to change the temperature on the screen of the display unit 12 (Step S108) and proceeds to Step S102 of M1 in FIG. 15 in order to acquire illuminance, temperature, and humidity.

After the illuminance, temperature, and humidity are acquired in Step S102, the humidity determining unit 39 determines whether the current humidity is within a comfortable humidity range suitable for sleep (Step S109). When the current humidity is within the comfortable humidity range (Yes in Step S109), the humidity determining unit 39 displays a humidity advice on comfortable sleep on the screen of the display unit 12 (Step S110) and ends the processing operation illustrated in FIG. 15. When the current humidity is not within the comfortable humidity range (No in Step S109), the humidity determining unit 39 displays a humidity advice to change the humidity on the screen of the display unit 12 (Step S111) and proceeds to Step S102 in order to acquire illuminance, temperature, and humidity.

When an operation of tapping the environment check button on the display screen is not detected (No in Step S101), the control unit 40 ends the processing operation illustrated in FIG. 15.

In the sleep environment determination process illustrated in FIG. 15, when the current illuminance detected by the illuminance sensor 18 of the portable terminal 1 is within the comfortable illuminance threshold value, the illuminance determining unit 37 displays an illuminance advice on comfortable sleep on the screen of the display unit 12. As a result, the user can see the illuminance advice and recognize that the current illuminance is comfortable for sleep.

When the current illuminance is not within the comfortable illuminance threshold value, the illuminance determining unit 37 displays an illuminance advice to adjust the current illuminance on the screen of the display unit 12. As a result, the user can see the illuminance advice and recognize that the current illuminance is not suitable for sleep.

When the current temperature detected by the temperature sensor 16 of the portable terminal 1 is within the comfortable temperature range, the temperature determining unit 38 displays a temperature advice on comfortable sleep on the screen of the display unit 12. As a result, the user can see the temperature advice and recognize that the current temperature is comfortable for sleep.

When the current temperature is not within the comfortable temperature range, the temperature determining unit 38 displays a temperature advice to adjust the current temperature on the screen of the display unit 12. As a result, the user can see the temperature advice and recognize that the current temperature is not suitable for sleep.

When the current humidity detected by the humidity sensor 17 of the portable terminal 1 is within the comfortable humidity range, the humidity determining unit 39 displays a humidity advice on comfortable sleep on the screen of the display unit 12. As a result, the user can see the humidity advice and recognize that the current humidity is comfortable for sleep.

When the current humidity is not within the comfortable humidity range, the humidity determining unit 39 displays a humidity advice to adjust the current humidity on the screen of the display unit 12. As a result, the user can see the humidity advice and recognize that the current humidity is not suitable for sleep.

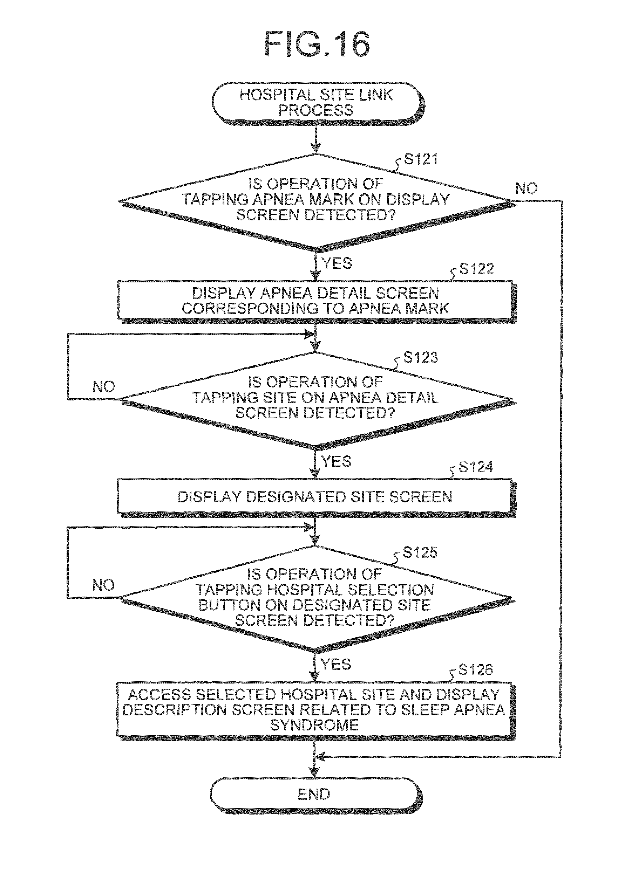

FIG. 16 is a flowchart illustrating an example of the processing operation of the processor 23 in the portable terminal 1 for a hospital site link process. The hospital site link process illustrated in FIG. 16 displays the detailed content of apnea on the screen when an operation of tapping the apnea mark 81 on the sleep log screen 70 is performed, and then leads the user to a hospital site.

In FIG. 16, the control unit 40 of the processor 23 determines whether the operation of tapping the apnea mark 81 on the sleep log screen 70 illustrated in FIG. 8 is detected (Step S121). When the operation of tapping the apnea mark 81 is detected (Yes in Step S121), the control unit 40 reads data for apnea corresponding to the apnea mark 81 from the sleep log region 22B and displays an apnea detail screen on the display unit 12 (Step S122). The apnea detail screen includes, for example, an apnea state between a snoring sound and a body motion, a schematic diagram illustrating the duration of the apnea state from the start time to the end time thereof, comment for apnea, and a designated site button used to access a designated site. The schematic diagram illustrates, for example, the apnea state between the breath sound and the body motion and the duration of the apnea state from the start time to the end time thereof.

The control unit 40 determines whether an operation of tapping the designated site button on the apnea detail screen is detected (Step S123). When the operation of tapping the designated site button is detected (Yes in Step S123), the control unit 40 accesses the designated site and displays a designated site screen on the screen of the display unit 12 (Step S124). In addition, the control unit 40 determines whether an operation of tapping a hospital selection button on the designated site screen is detected (Step S125).

When the operation of tapping the hospital selection button on the designated site screen is detected (Yes in Step S125), the control unit 40 displays a description screen for sleep apnea syndrome in the selected hospital site on the display unit 12 (Step S126). Then, the control unit 40 ends the processing operation illustrated in FIG. 16.

When the operation of tapping the apnea mark 81 on the sleep log screen 70 is not detected (No in Step S121), the control unit 40 ends the processing operation illustrated in FIG. 16. When the operation of tapping the designated site button on the apnea detail screen is not detected (No in Step S123), the control unit 40 proceeds to Step S123 in order to determine whether the operation of tapping the designated site button is detected. When the operation of tapping the hospital selection button on the designated site screen is not detected (No in Step S125), the control unit 40 proceeds to Step S125 in order to determine whether the operation of tapping the hospital selection button is detected.

In the hospital site link process illustrated in FIG. 16, when the operation of tapping the apnea mark 81 on the sleep log screen 70 is detected, the control unit 40 displays the apnea detail screen on the display unit 12. As a result, the user can see the apnea detail screen and recognize detailed information on the apnea state.

When the operation of tapping the designated site button on the apnea detail screen is detected, the control unit 40 displays the designated site screen on the display unit 12. As a result, the user can see the designated site screen and recognize the hospital which specializes in sleep apnea syndrome.

When the operation of tapping the hospital selection button on the designated site screen is detected, the control unit 40 displays the description screen related to the sleep apnea syndrome provided by the selected hospital on the display unit 12. As a result, the user can see the description screen for the hospital and recognize the content of sleep apnea syndrome.

In this embodiment, when a sound in which a component is similar at each frequency is periodically detected from the recorded data during sleep, the breath determining unit 31 determines the detected sound to be a breath sound. When a silent interval is detected between the determined breath sound and a body motion, the apnea determining unit 33 determines the silent interval to be an apnea state. As a result, since breathing timing is used, it is possible to determine a state which can be considered as the apnea state between the breath sound and the body motion with high accuracy.

In this embodiment, when a sound in which a component is similar at each frequency is periodically detected from the recorded data during sleep, the breath determining unit 31 determines the detected sound to be a breath sound. When the sound pressure level of the determined breath sound is greater than the first level threshold value, the snore determining unit 32 determines the detected sound is a snoring sound. As a result, even when noise corresponding to snoring between successive breath sounds is generated, the noise is not determined to be a snoring sound, which makes it possible to prevent an error in the determination of the snoring sound.

In this embodiment, when the silent interval is detected between the determined snoring sound and the body motion, the apnea determining unit 33 determines the silent interval to be the apnea state. As a result, since snoring timing is used, it is possible to determine a state which can be considered as the apnea state between the snoring sound and the body motion with high accuracy.

In this embodiment, the control unit 40 displays the sleep log screen 70 based on the sleep log on the screen of the display unit 12. As a result, the user can see the sleep log screen 70 and recognize, for example, the number of body motions, the number of snores, and the number of apneas, in addition to information about sleep. In addition, the user can recognize a sleep advice on the sleep log.

Furthermore, the user can tap the apnea mark 81 on the sleep log screen 70 to recognize the apnea detail screen at that time. The user can see the sleep advice on the sleep log screen 70 and recognize the advice on the current sleep log. The user can tap the designated site button on the sleep log screen 70 to simply access the designated site. After accessing the designated site, the user can tap the hospital selection button on the designated site screen, see the description screen related to sleep in the selected hospital site, and recognize symptoms related to sleep. As a result, the user can be aware of, for example, sleep apnea syndrome according to the content of the sleep log.

In the above-described embodiment, the portable terminal 1, such as a smart phone, is given as an example. However, for example, mobile phones, portable game terminals, tablet terminals, and portable terminals without a communication function may be used as the portable terminal.

In the above-described embodiment, when the operation of tapping the calendar button is detected, the calendar screen is displayed on the display unit 12. At that time, a monthly rank of daily sleep logs is displayed on the calendar screen.

In the above-described embodiment, the sleep log screen 70 is displayed on the display unit 12 on a daily basis. However, for example, the number of snores, the number of apneas, or the number of body motions per week or month may be counted and the sleep log screen may be displayed on the display unit 12 on a weekly basis or a monthly basis.

In the above-described embodiment, both the acoustic frequency component when a body motion is made and the acceleration component when the body motion is made are used to determine whether there is a body motion. However, one of the acoustic frequency component and the acceleration component may be used to determine whether there is a body motion.

Each component of each unit illustrated in the drawings is not necessarily physically configured as illustrated in the drawings. That is, the detailed configuration of the distribution and integration of each unit is not limited to that illustrated in the drawings, but a portion of or the entire unit may be functionally or physically distributed or integrated in an arbitrary unit according to, for example, various kinds of loads or usage conditions.

All or some of various kinds of processing functions performed by each apparatus may be executed on a central processing unit (CPU) (or a micro·computer, such as a micro processing unit (MPU) or a micro controller unit (MCU)). In addition, all or some of various kinds of processing functions may be executed on a program which is analysed and executed by a CPU (or a micro·computer, such as an MPU or an MCU) or hardware including a wired logic.

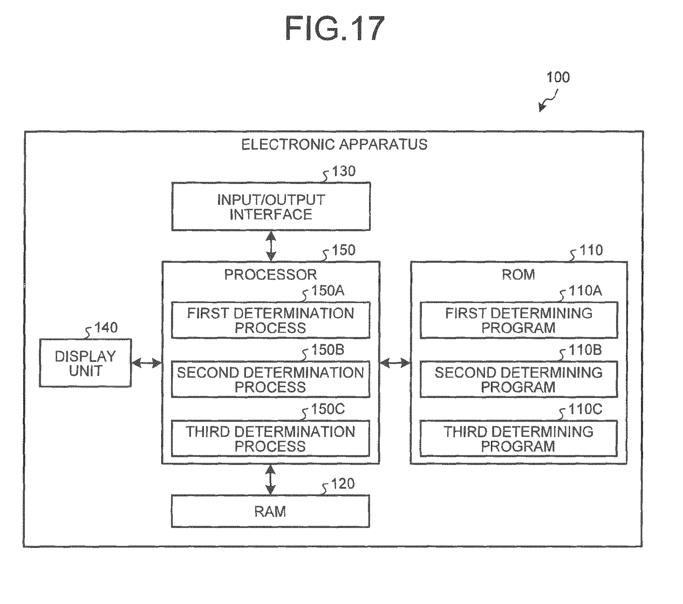

However, various kinds of processes described in this embodiment may be implemented by executing a program which is prepared in advance in an electronic apparatus. Next, an example of the electronic apparatus which executes a program having the same function as that in the above-described embodiment will be described. FIG. 17 is a diagram illustrating an example of the electronic apparatus which executes an apnea determining program.

An electronic apparatus 100 which executes the apnea determining program illustrated in FIG. 17 includes a ROM 110, a RAM 120, an input/output interface 130, a display unit 140, and a processor 150.

The ROM 110 stores the apnea determining program having the same function as that in the above-described embodiment in advance. However, the apnea determining program may not be stored in the ROM 110, but may be recorded on a recording medium which can be read by a drive (not illustrated). Examples of the recording medium may include portable recording media, such as CD-ROMs, DVD disks, USB memories, and SD cards, and semiconductor memories, such as flash memories. Examples of the apnea determining program include a first determining program 110A, a second determining program 110B, and a third determining program 110C, as illustrated in FIG. 17. The programs 110A, 110B, and 110C may be appropriately integrated or distributed.

The processor 150 reads the programs 110A, 110B, and 110C from the ROM 110 and executes each of the read programs on the RAM 120. Then, as illustrated in FIG. 17, the programs 110A, 110B, and 110C executed by the processor 150 function as a first determination process 150A, a second determination process 150B, and a third determination process 150C, respectively.

When a sound in which a component is similar at each frequency is periodically detected from the collected sounds, the processor 150 determines the detected sound to be a breath sound of an object person. The processor 150 determines whether a body motion of the object person is detected after the determined breath sound. When the body motion is detected, the processor 150 determines whether a silent interval is detected between the breath sound and the body motion. When the silent interval is detected, the processor 150 determines the silent interval to be an apnea state. As a result, it is possible to determine a state which can be considered as the apnea state with high accuracy.

## Claims

1. An apnea determining program that causes a processor(23) of an electronic apparatus(1) to perform:
when having detected sounds having similar frequency components periodically in picked-up sounds, first determining(31) the detected sounds to be breath sounds of an object person;
second determining(34) whether a body motion of the object person is detected after the breath sounds are determined at the first determining;
third determining(33) whether a silent interval is detected from a period of time between timings of the breath sound determined at the first determining and the body motion detected by the second determining when the body motion is detected; and
fourth determining(33) the silent interval to be an apnea state when the silent interval is detected.

2. The apnea determining program according to claim 1,
wherein the second determining(34), when the sound pressure of the determined breath sounds is greater than a predetermined threshold value, includes fifth determining the breath sound to be a snoring sound and sixth determining whether the body motion of the object person is detected after the snoring sound is determined, and
the fourth determining(33), when the body motion is detected, includes determining whether the silent interval is detected from a period of time between timings of the snoring sound determined at the fifth determining and the body motion detected by the sixth determining.

3. An apnea determining device(1) comprising:
a first determining unit(31) that, when having detected sounds having similar frequency components periodically in picked-up sounds, determines the detected sounds to be breath sounds of an object person;
a second determining unit(34) that determines whether a body motion of the object person is detected after the breath sounds are determined at the first determining;
a third determining unit(33) that determines whether a silent interval is detected from a period of time between timings of the breath sound determined at the first determining unit and the body motion detected by the second determining unit when the body motion is detected; and
a fourth determining unit(33) that determines the silent interval to be an apnea state when the silent interval is detected.

4. An apnea determining method that is performed in an electronic apparatus(1), the method comprising:
when having detected sounds having similar frequency components periodically in picked-up sounds, first determining the detected sounds to be breath sounds of an object person;
second determining whether a body motion of the object person is detected after the breath sounds are determined at the first determining;
third determining whether a silent interval is detected from a period of time between timings of the breath sound determined at the first determining and the body motion detected by the second determining when the body motion is detected; and
fourth determining the silent interval to be an apnea state when the silent interval is detected.
